Europäisches Patentamt

European Patent Office

Office européen des brevets

(19)

(11) Publication number: **0 061 843**

A2

(12) **EUROPEAN PATENT APPLICATION**

(21) Application number: **82301097.0**

(22) Date of filing: **04.03.82**

(51) Int. Cl.³: **A 61 F 7/00**

(30) Priority: 28.03.81 JP 44179 81
30.07.81 JP 118455/81
06.08.81 JP 122393/81
06.08.81 JP 122394/81
06.08.81 JP 122395/81
06.08.81 JP 122396/81

(43) Date of publication of application:
06.10.82 Bulletin 82/40

(84) Designated Contracting States:
DE FR GB IT NL

(71) Applicant: Nakamatsu, Yoshiro
1-10-309, 5-chome, Minami Aoyama Minato-ku
Tokyo(JP)

(72) Inventor: Nakamatsu, Yoshiro
1-10-309, 5-chome, Minami Aoyama Minato-ku
Tokyo(JP)

(74) Representative: Shaw, Laurence
George House George Road
Birmingham, B15 1PG(GB)

(54) Apparatus for increasing brain activity.

(57) Apparatus to improve the brain activity of a person, comprises means for cooling the head and for warming the feet. The apparatus may take a variety of forms ranging from chairs, beds, and mattresses, and may be incorporated in a car driver's seat. Apparatus is also provided to measure the rate of brain activity.

FIG.1

EP 0 061 843 A2

0061843

## APPARATUS FOR INCREASING
## BRAIN ACTIVITY

The invention relates to apparatus for increasing the brain activity of a human person.

The invention is based upon the discovery that if the head of a person is cooled at the same time as the feet are warmed, the mental activity of the person is increased.

According to one aspect of the invention there is provided apparatus for the purpose specified <u>characterised by</u> means for cooling the head of a person and means for warming the sole of person.

Preferably the means is incorporated in an article of furniture such as a chair, desk or bed where the person can be when it is appropriate to raise the level of brain activity. The item of furniture may be in a house or in a vehicle.

The cooling and heating means may be separate or interconnected. My investigations have shown that unless both cooling and heating take place at the same time, the brain activity is not increased, as the evaluations reported below show.    It is much preferred to cool the head to a temperature of about 0$^{O}$C to 20$^{O}$C, and to warm the feet to about 25$^{O}$C to 60$^{O}$C.    In this way the temperature difference between the head and the feet can range from about 5$^{O}$C to about 60$^{O}$C.

The invention also includes apparatus for measuring the brain activity comprising means for receiving a part of the person whose brain activity is to be measured e.g. a finger, means for radiating light through the body part, means for receiving the light transmitted through the body part and converting it into an electrical signal and means for indicating the magnitude of the signal. Preferably the body part has blood vessels and is a finger.

In order that the invention may be well understood, it will now be described by way of example, with reference to the accompanying diagrammatic drawings in which:

Figure 1 is a schematic view illustrating the principle of this invention;

Figure 2 is a side elevation, partly in section, of a chair of the invention and Figure 3 is a plan view thereof;

Figure 4 is a circuit diagram of a coolant system incorporated in the chair of Figures 2 and 3;

Figures 5 to 9 are sectional view of different

0061843

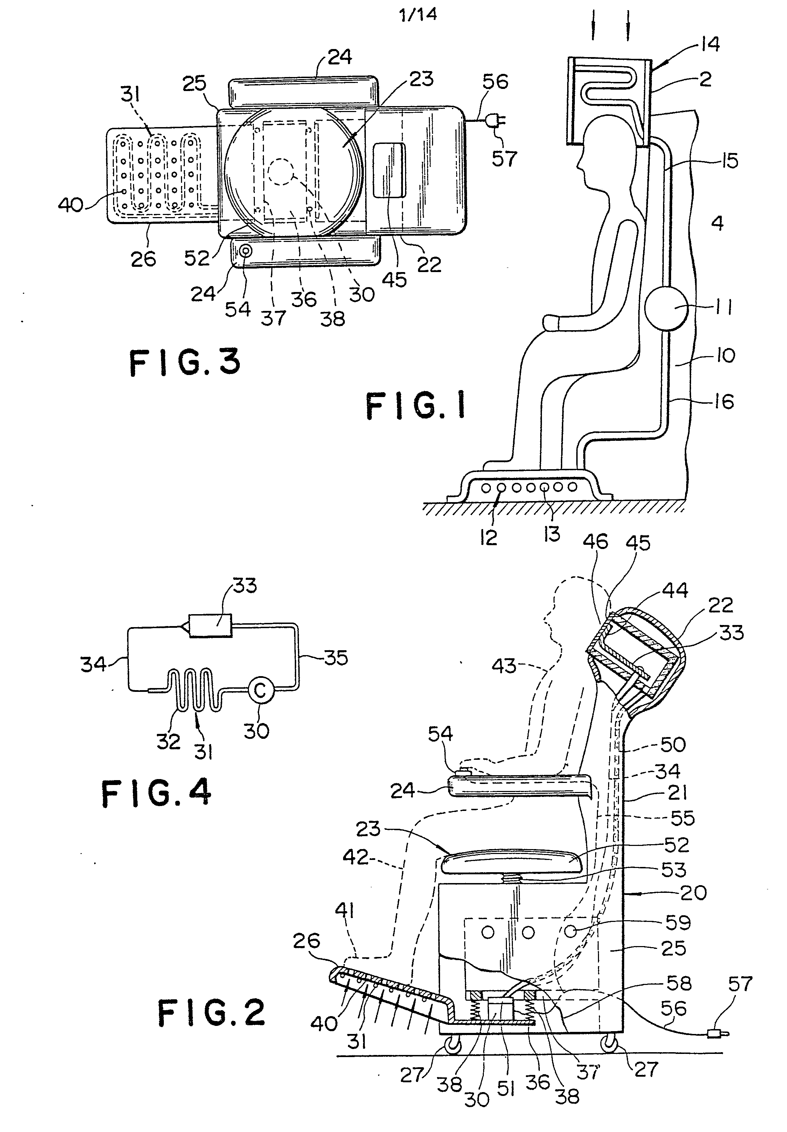

head rests in the chair of Figures 2 and 3;

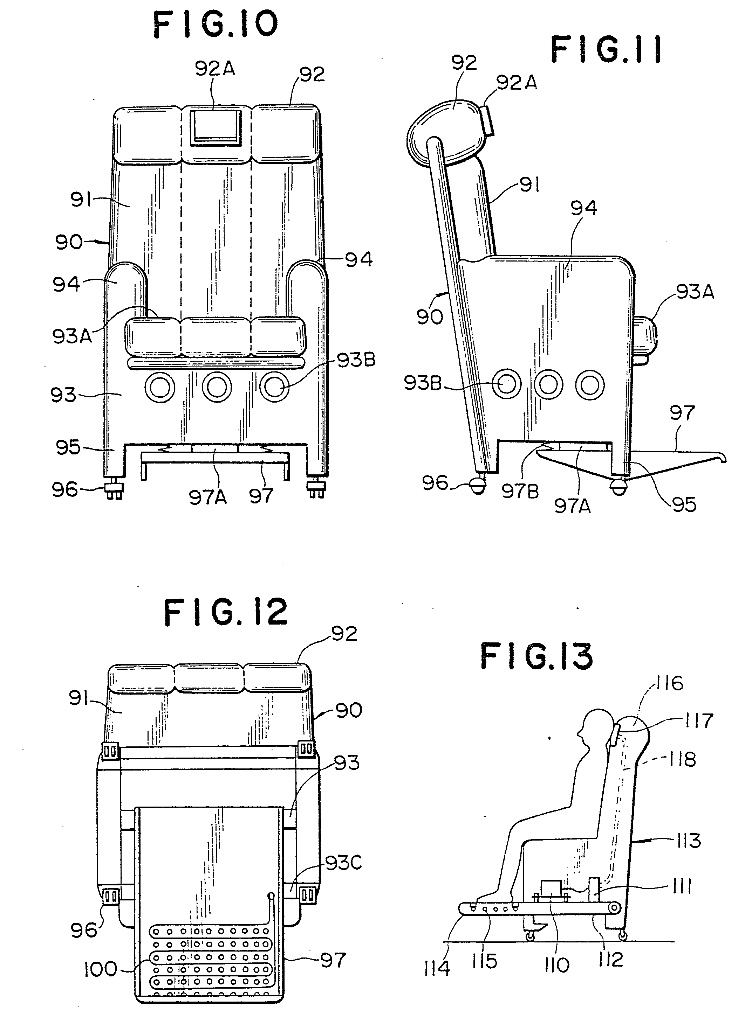

Figures 10 to 12 are respectively front elevation, side elevation and underside view of another chair of the invention;

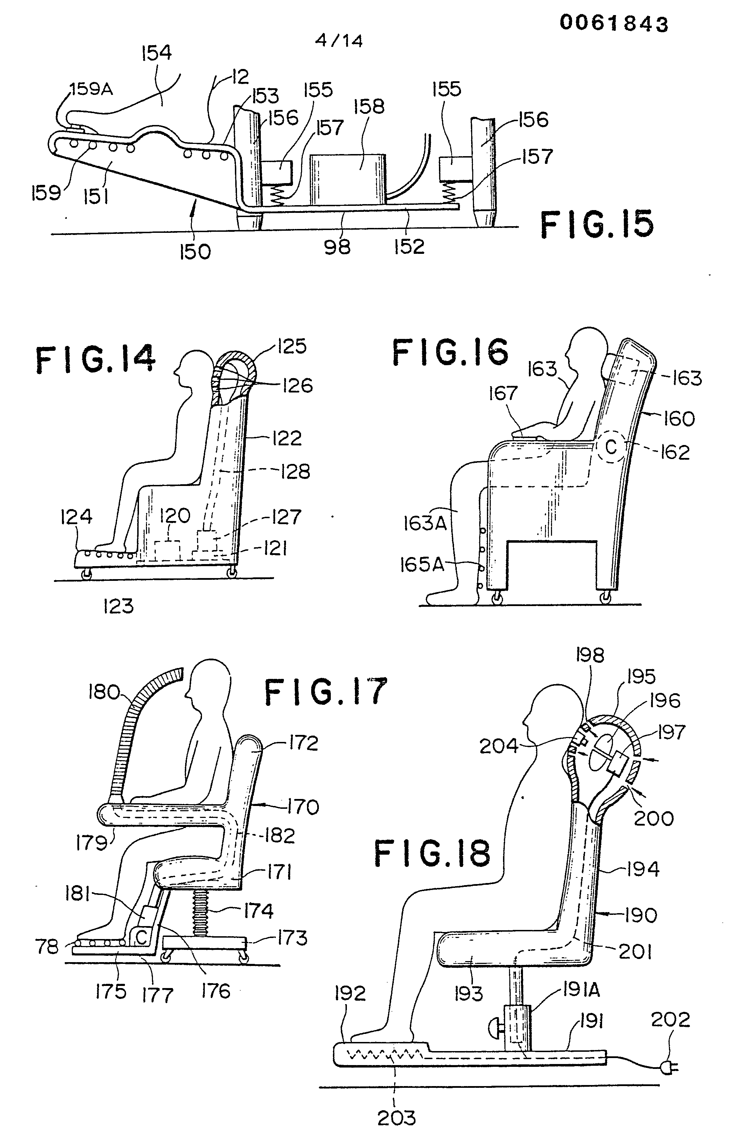

Figures 13 and 14 are each a side view of other chairs of the invention;

Figure 15 is a fragmentary side view of a foot rest for use in a chair of the invention;

Figures 16 and 17 are side elevations of other chairs of this invention;

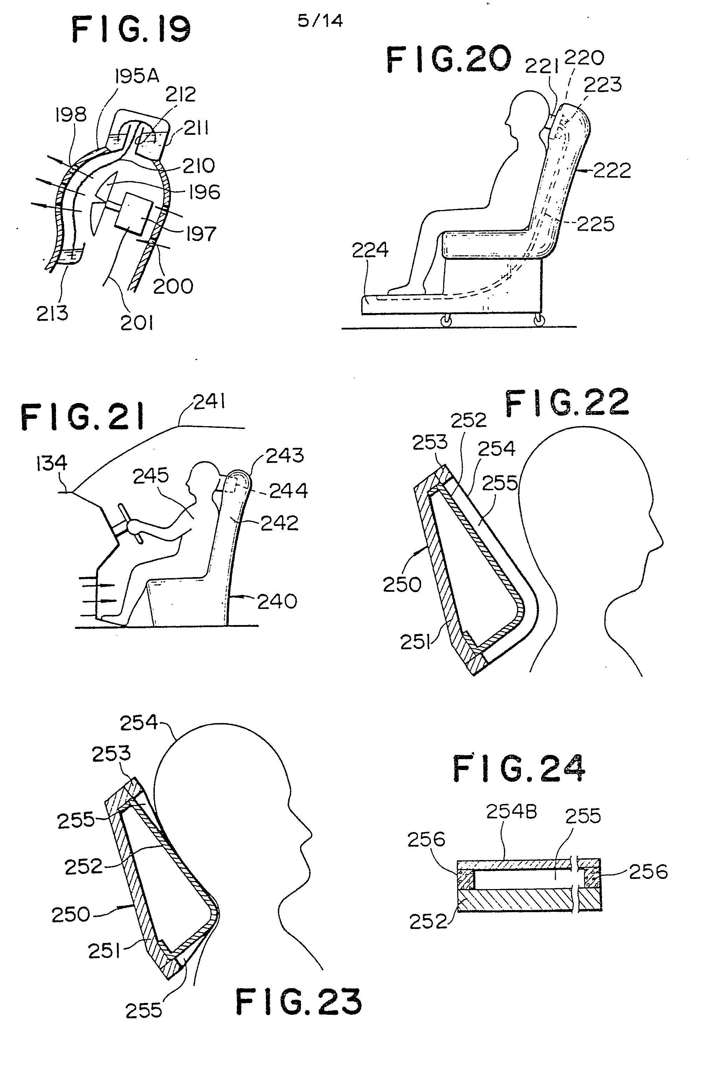

Figure 18 is a side elevation of another chair and Figure 19 is a fragmentary section of a modification thereof;

Figure 20 is a side elevation of another chair of the invention;

Figure 21 is a schematic side view of a chair of the invention in an automobile;

Figure 22 is a side section of a head rest of the invention, Figure 23 shows the head rest in use and Figure 24 is a sectional view thereof;

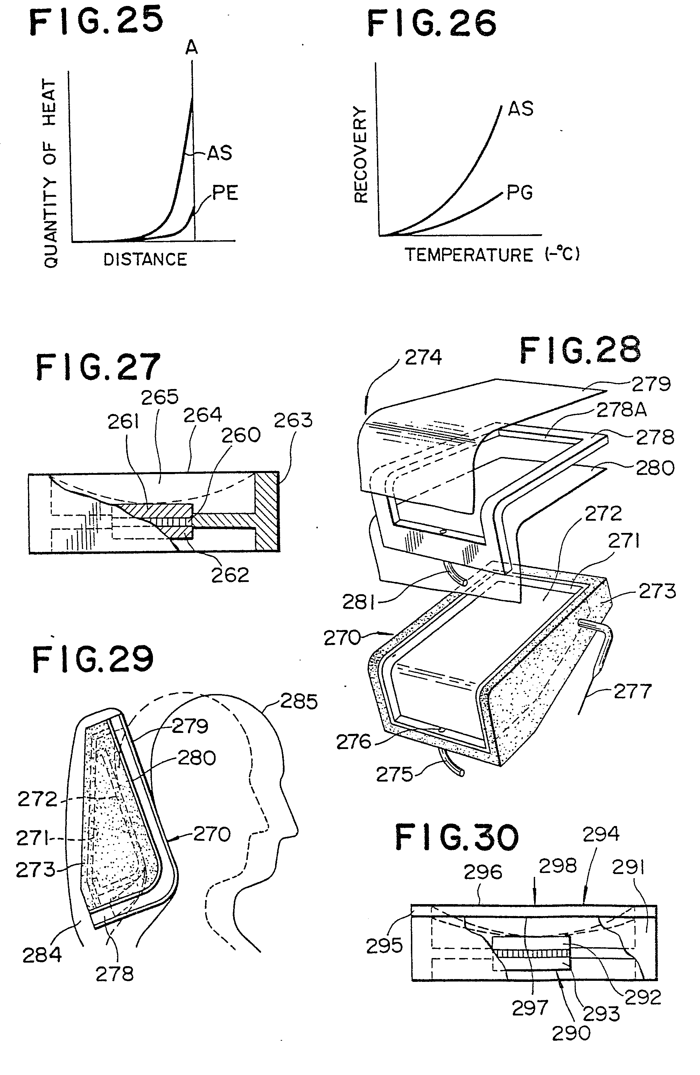

Figures 25 and 26 are graphs showing properties of cover materials useful in the head rest of this invention;

Figure 27 is a top view, partly in section, of another head rest of the invention;

Figure 28 is an exploded perspective view of another head rest of the invention;

Figure 29 is a side elevation showing the head rest of Figure 28 in use;

Figure 30 is a top view, partly in section, of another head rest of the invention;

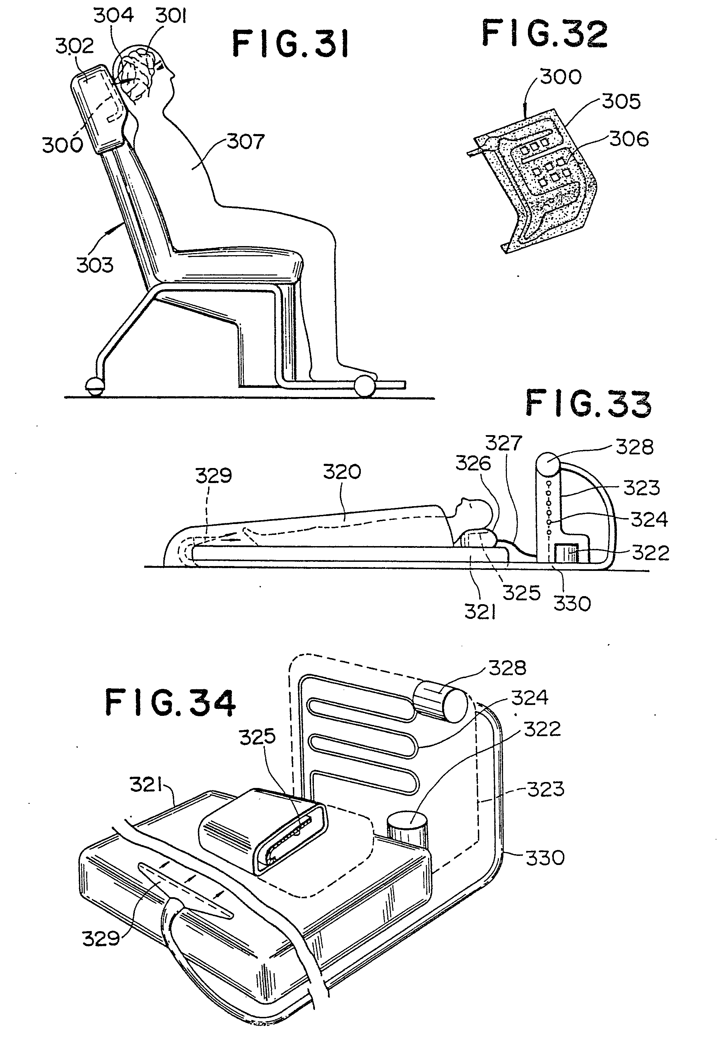

Figure 31 is a schematic side view of another chair of the invention and Figure 32 is a perspective view of a cooling section used in the chair of Figure 31;

Figure 33 is a side elevation view of a mattress of the invention and Figure 34 is a perspective view, partly in section, of apparatus shown in Figure 33;

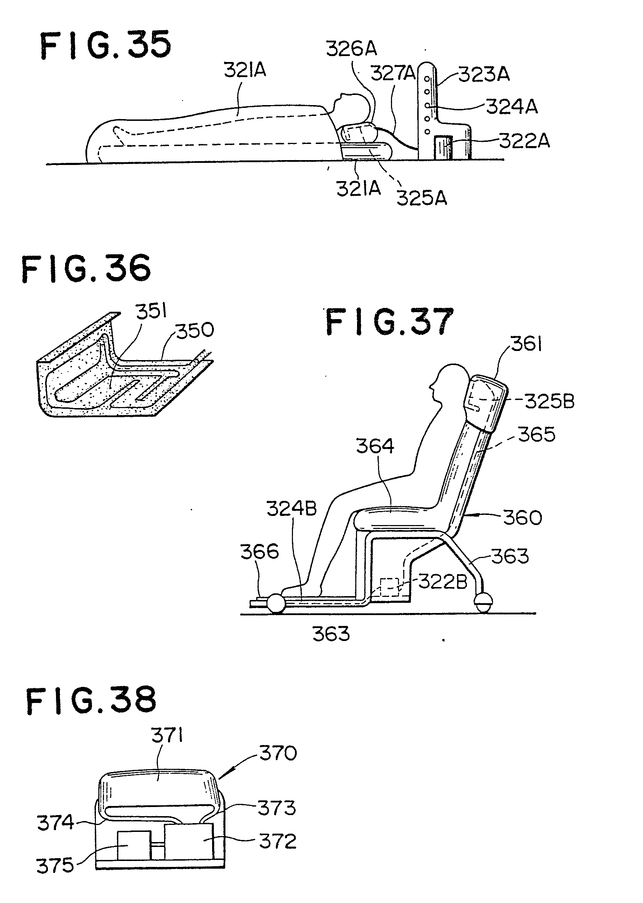

Figure 35 is a side elevation view of another apparatus of the invention, and Figure 36 is a perspective view of the cooling member thereof;

Figure 37 is a side elevation of another chair of the invention;

Figure 38 is a front elevation of a pillow of the invention;

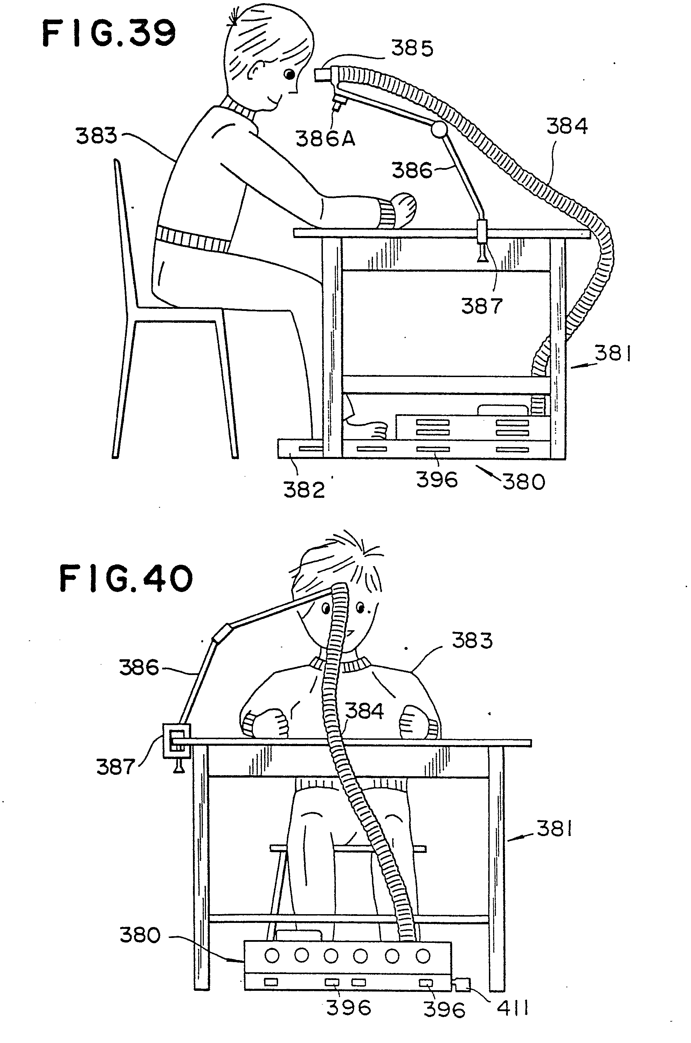

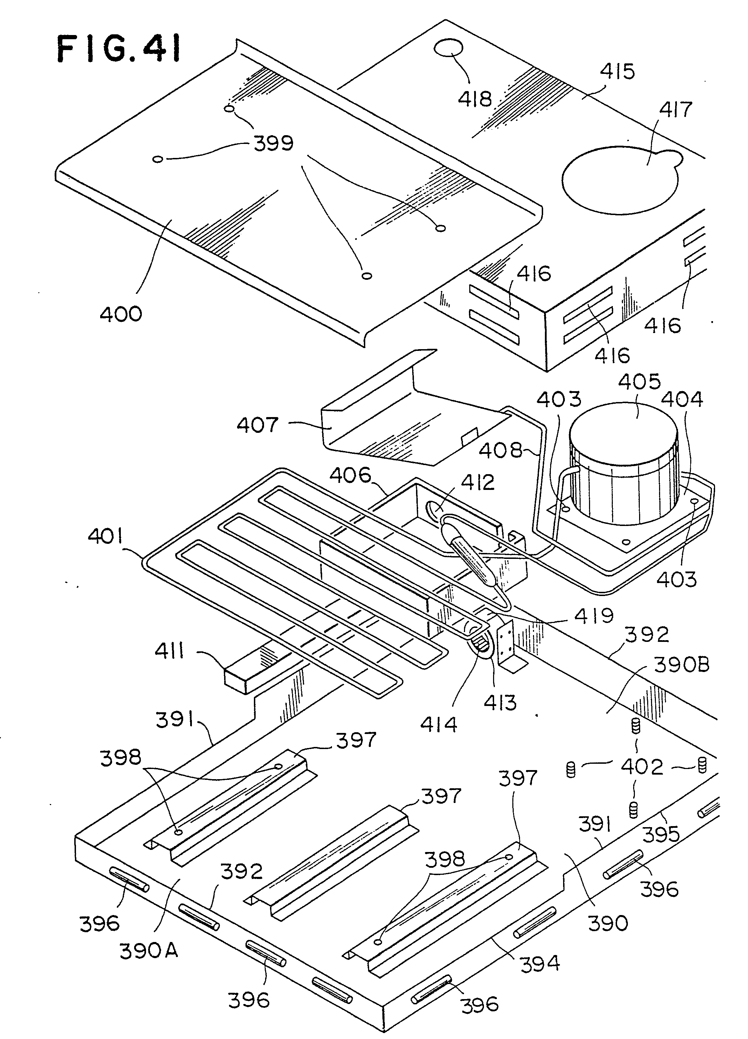

Figure 39 is a side elevation of a student desk of the invention and Figure 40 is a front elevation thereof;

Figures 41 to 47 show details of parts used in the desk of Figures 39 and 40;

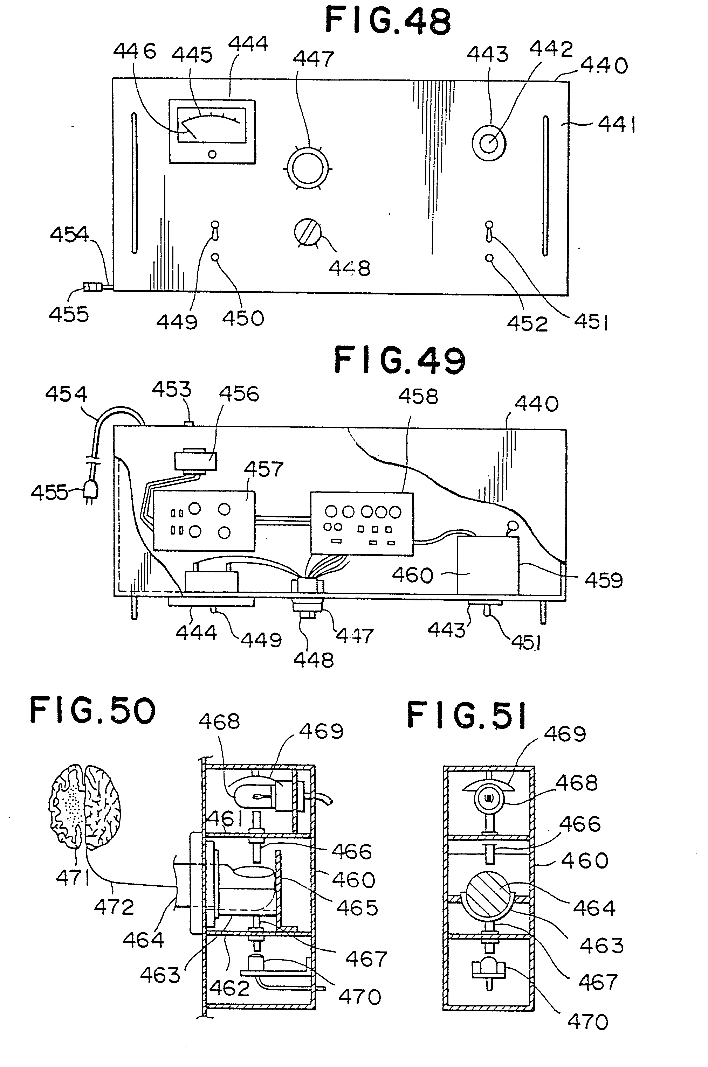

Figure 48 is a front elevation of an apparatus of the invention useful for measuring brain power and Figure 49 is a top view, partly in section thereof;

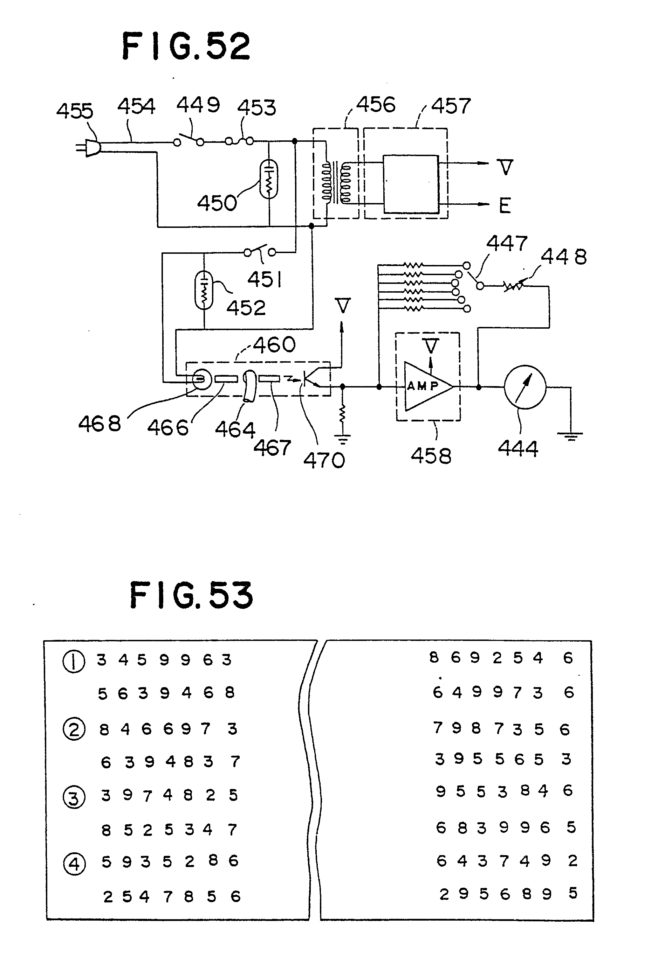

Figures 50 and 51 are sectional views showing detail of the apparatus of Figures 48 and 49;

Figure 52 is a circuit diagram incorporating the apparatus of Figure 48; and Figure 53 is a test paper for testing the measuring apparatus of Figure 48.

The apparatus of Figure 1 comprises a chair 10 having a foot rest.   A compressor 11 housed in the chair back is connected below by a pipe 16 to a condenser 12 comprising a convoluted pipe 13 in the foot rest and above via a pipe 15 to an evaporator 14 including a cylindrical housing to receive at least a portion of the head of a person.   When a person sits in the chair 10, his head is received in the cooling cylinder and his feet are placed on the warmed foot rest and the combined effects will result in an increase in brain activity of the person.

The chair 20 of Figures 2 to 4 comprises a back 21, a head rest portion 22, a seat portion 23, armrests 24, a lower box-like support 25 and a foot rest portion 26. Casters 27 are mounted on the chair 20.   A cooling-warming device shown in Figure 4 is incorporated in the chair 20 and comprises an electrically driven compressor 30, a condenser 31 comprising a convoluted heat-radiation pipe 32 which is connected with the compressor 30 downstream thereof, an evaporator 33 connected to the outlet of the condenser 31 via a capillary pipe 34 and a pipe portion 35 connecting the outlet of the evaporator 33 with the inlet of the compressor 30.    A coolant such as FREON (registered trade mark) may be present.

The compressor 30 is spring-mounted on a plate 36 which is suspended from a cross member 37 by springs 38 and which has a forward extension defining a perforated foot rest 26.    The condensor 31 is mounted on the underside of the foot rest 26.    Air is heated by the condensor 31 and then passed upwardly through the perforations 40 in the foot rest 25 to warm the feet 41 and legs 42 of a person 43 sitting in the chair 20.    The box 44 is shaped to receive the back of the person's head and the surface has a cover sheet 45 of a heat-conductive and flexible material such as metal sheet or plastic sheet. In Figure 2 the evaporator 33 is a cooling plate of an L-shaped section with the smaller leg of the L located opposite the inner face of the cover sheet 45.    When the person's head 46 bears on the cover sheet 45, the latter is urged inward into contact with the smaller leg of the cooling plate so that the person's head 46 is cooled by the evaporator 33 through the cover sheet 45.    The evaporator 33 is thermally insulated relative to atmosphere so that it will not be overloaded.    Waste water in the box 44 is removed via a drain pipe 50 to an evaporating dish 51 mounted on the compressor 30.    The seat portion 23 includes a seat 52 adjustable on a threaded shaft 53.

One of the armrests 24 has a power switch 54 for controlling the motor (not shown) of the compressor 30 via a line 55 and connected with a power supply (not shown) through a line 56 having a plug 57.   ·The lower sides 25 of the chair 20 are provided with sidewalls 58 for the compressor section 30 and having vents 59.

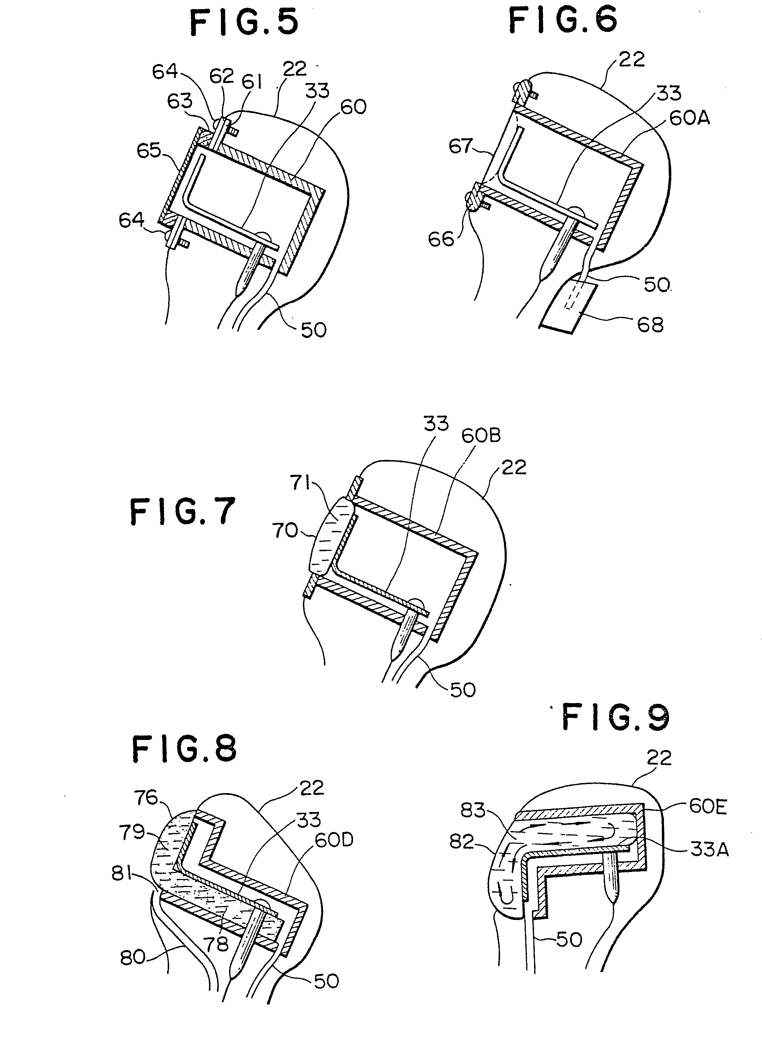

Figures 5 to 9 show various types of the cooling section for the head rest 22 of the chair 20.   In Figure 5, the cooling section comprises a thermally insulated box 60 having a forward flange 61.   A flange 62 of resilient frame 63 is screwed by screws 64 to the flange 61. The frame 63 has a head-engaging metal or plastics plate 65 attached to the frame and when the person bears against the head-engaging plate 65 the plate 65 is urged into contact with the cooling plate 33.

In figure 6, a flexible plastics film 67, e.g. polyvinyl chloride is clamped to the front of the box 60A by clamps 66 and is spaced slightly away from the cooling plate 33.   The drain pipe 50 is connected to a vessel 68 which is detachably mounted at the lower end of the head rest 22.   In Figure 7, a flexible bag 70 is mounted on the front of a thermally insulating box 60B but in contact with the cooling plate 33.   The vessel 70 contains a liquid 71 having a low freezing point, e.g. 50% aqueous solution of ethylene glycol, propylene glycol, agar
solution or the like.    The vessel 70 may be replaced by
a cushion and a bag containing a heat-conductor including
aluminium wires or steel wool.    In Figure 8, a
substantially L-shaped ceramic element extends along the
cooling plate 33 and includes a longer leg portion 78
extending into the box 60D along the corresponding leg
portion of the cooling plate 33 and a shorter leg portion
engaged by the corresponding shorter leg of the cooling
plate 33 and serving as a lid for the open end of the box
60D.    The device includes a drain pipe 80 with an inlet
81 for receiving droplets of water which condense on the
ceramic member 76.

The box 60E of Figure 9 includes a cooling plate
33A disposed parallel to an inverted box 60E.    A plastic
bag 82 containing antifreeze 83 extends along the entire
upper surface of the cooling plate 33A and into the box
60E.    When the bag 82 is engaged by the head of the
person, the anti-freeze liquid 83 is circulated in the
bag 82 to cool the person's head more effectively.
Condensed water accumulates at the bottom of the shorter
leg portion of the box 60E and is drained off via the
drain pipe 50.

Figures 10 to 12 show a chair 90 having a back
91, a head rest 92, a base 93 supporting the back 92 and having a seat 93A and armrests 94, legs 95 supporting the base 93 and having casters 96 and a foot rest 97 extending outwardly from between the forward legs 95 of the base 93 and suspended from the cross beams 93C of the base 93 by springs 97B.   A cooling and warming circuit comprises a compressor 97A mounted on the foot rest 97 below the base 93, an evaporator connected to the compressor 97A with a portion 92A exposed at the head rest 92, and a convoluted warming condenser pipe 100 below the foot rest 97.   This foot rest 97 has vent holes 98 to allow upward movement of air warmed at the condenser pipe 100 and the base 93 has side vents 93B to allow air heated at the compressor 97A to escape to atmosphere.

Figure 13 shows another chair in which a cooling and warming circuit includes a compressor 110 and an evaporator 111 mounted on a base plate 112 which is pivotally mounted on a chair 113 at the rearward end and suspended from the bottom of the chair 113 at the intermediate part of the base plate 112 by springs (not shown). The forward portion of the base plate 112 extends forward beyond the chair 113 to form a foot rest 114 incorporating a condenser 115.   The chair 113 has a head rest on which is mounted a cooling terminal 117 made of heat-conductive material such as copper or aluminium.   The cooling terminal 117 is connected to the evaporator 111 by a heat pipe 118.

In the chair of Figure 14, a cooling and warming circuit comprises a compressor 120 and an evaporator 121 within the bottom of a chair 122. This circuit also includes a condenser 123 mounted within a foot rest 124 of the chair 122. The chair 122 has a head rest 125 having forward openings 126 connected to fan 127 via a pipe 128. The fan 127 is mounted on the evaporator 121 to supply cooled air to the head rest 125 through the pipe 128 towards the user's head.

In the embodiment of Figure 15 a foot rest 150 includes a forward portion 151 and a rearward portion 152 separated by a hump 153 shaped to complement the arches of the person's feet 154. The rearward portion 152 of the foot rest 150 is suspended from transverse studs 155 on the legs 156 of a chair (not shown) by springs 157, and support a compressor 158. The vibration from the compressor 158 serves to massage the person's feet 154 on the forward portion 151 of the foot rest 150 but is not transmitted to the chair. The forward portion 151 includes a condenser 159 and a foot actuated power switch 158A.

Figure 16 shows a chair 160 having a back 161 including a compressor 162 to massage the back of a person 163.   A condenser 165A is located forwardly of the chair legs and behind the legs 163A of the person 163 sitting in the chair 160.

Figure 17 shows a chair 170 including a back 172 and a seat portion 171 screwed to a base portion 173 by a threaded shaft 174.   A foot rest 175 is suspended from a substantailly vertical plate 176.   A compressor and evaporator unit 177 is mounted at the rear of the foot-rest portion 175 and a condenser 178 is mounted at the front. Armrests 179 extend forwardly from the back 172 of the seat portion 171.   One of the armrests 179 has an upwardly extending duct forward 180.   The duct 180 has one open end near the forehead of a person sitting on the chair 170 with the other open end connected to the compressor and evaporator unit 177 through a fan 181 via a pipe 182 which passes through the seat portion 171, the lower part of the back 172 and the one armrest 179.   Air is cooled at the compressor and evaporator unit 177 and conducted to the forehead of the person through the pipe 182 and duct 180.

The chair 190 of Figure 18 comprises a base portion having a foot rest 192, a seat portion 193 connected with the base 191 by a screw thread 191A, a back 194 integral with the seat 193, and a head-rest portion 195 formed on the top of the back 194.   A fan 196 driven by an electric motor 197 is present in the head rest
portion 195. Openings 198 are formed in the forward
wall of the head rest 195 in front of the fan 195 and
openings 200 are formed in the rearward wall of the
head rest 195 behind the motor 197. As the fan 196 is
driven by the motor 197, air is drawn into the head rest
195 through the rearward openings 200 and then blown to
the head of a person sitting on the chair 190 through the
forward openings 198. The electric motor 197 is
connected to a power supply (not shown) through an
electric line 201 having a plug 202. The electric line
120 is electrically connected to an electric heater 203
which is mounted in the foot rest 192. The forward wall
of the head rest 195 also has a power switch 204 for
controlling the electric motor 200 and heater 203.

The embodiment of Figure 19 is similar to that
of Figure 18 except that a sheet of roughly woven cloth
210 is located in the head rest 195A to block the flow
of air from the fan 196. The cloth 210 is immersed at
the upper end in the water contained in a vessel 211 which
is mounted on the top of the head rest 195A. The vessel
211 has a central open-ended tube portion 212 which
extends inwardly of the vessel 211 for conducting the one
end of the cloth 210 to the water in the vessel. The
other or lower end of the cloth 210 is received into another vessel 213 mounted on the inner wall of the head
rest 195A at the lower end thereof.  Air can also be
cooled by evaporation on the wet cloth 210.

Figure 20 shows an embodiment which utilises
an electronic device based on Peltier effect.   This
device comprises a thermoelement 220 having a cold section
221 disposed in the head rest of a chair 222 against the
head of a person sitting on the chair 222 and a hot
section 223 which is located in the head rest of the chair
222 and connected to a warming section in the foot rest 224
via a heat pipe 225.

Figure 21 shows a seat of this invention in an
automobile 241.   The seat 240 has a back 242 and a head
rest 243 which includes a cooling means 244.   The feet of a
driver  245 can be warmed by the warmed air from the
conventional heater (not shown) of the automobile 241, as
shown by arrows.

Figures 22 and 23 show a head rest which
includes a cooling section, for example, the evaporator
section of the cooling and warming circuit utilising a
coolant such as FREON.   The head rest 250 comprises a
thermally insulated casing 251 e.g. foamed polyethylene,
and a cooling plate 252.   A frame 253 is mounted on the outer edge of the casing 251 and is covered by a flexible sheet 254 to cover the cooling plate 252.    An air layer 255 is thus formed between the cooling plate 252 and the flexible sheet 254 so that the former is thermally insulated.    When a person rests his head on the head rest 250, the flexible sheet 254 is deformed inwardly to bring the person's head into engagement with the cooling plate 252 through the flexible sheet 254.    The flexible sheet 254 is made of any suitable elastomeric, heat conductive and low-temperature resistance material such as a silicon rubber sheet containing metal powder therein, for example aluminium powder.


Figure 24 shows a cover plate 254 comprising a metal sheet 254B mounted on the cooling plate 252 through cushion members 256 of any elastomeric material such as polyurethane foam.    The sheet 125A can be urged inwardly parallel to the cooling plate 252.    A polyester film can be used in place of the metal sheet 254A.


Figure 25 is a graph indicating the relationship

between the quantity of heat transmitted from the cooling plate to the human head through the flexible sheet and the distance from the flexible sheet to the cooling plate, for different materials.    The vertical axis indicates the quantity of heat while a horizontal axis represents the
distance.   The vertical line A shows a position in which
the sheet is in contact with the cooling plate.   Curve PE
is for a polyester sheet while curve AS is for a silicon
rubber sheet containing aluminium powder.   This graph
shows that the silicon rubber sheet is a better heat-
conductor than the polyester sheet.   Polyvinyl chloride,
polyethylene and like sheets may all be used in this ·
invention.

Figure 26 is a graph indicating the relationship
between the recovery and the temperature of different
materials.   The vertical axis indicates the recovery
while a horizontal axis represents the temperature.   Curve
AS is for the silicon rubber sheet containing the aluminium
powder while curve PG is for polyvinyl chloride or rubber.
This graph shows that the silicon rubber sheet recovers
quickly at lower temperature.

Figure 27 shows a head rest in which an electronic
device utilising Peltier effect is used as a cooling and
warming system.   The device comprises a thermoelement 260
having a cold section 261 and a hot section 262.   The
theremoelement 260 is mounted in a heat-insulated frame 263
with the cold section 261 facing a flexible sheet 264
mounted on the frame 263 across the mouth thereof with an
air gap 265.

Figures 28 and 29 show a head rest 270 comprising an open casing 271 formed of plastic sheet and housing a cooling element 272 e.g. an evaporator with thermally insulating polyethylene foam walls 273 covering the outer walls of the casing 271 and to be engaged by the head of a person. The casing 271 includes a drain pipe 275 connected to a drain port 276 formed in the casing wall. The cooling element 272 housed in the casing 271 includes a capillary pipe 277 extending outwardly through the casing wall and the thermally insulating wall and connected with a compressor (not shown). The lid 274 is of substantially L-shaped form corresponding to the profile of the opening in the casing 271 and comprises an L-shaped frame 278 having an opening 278A, an outer flexible sheet 279 attached to the outer face of the frame 278, and an inner flexible sheet 280 mounted on the inner face of the frame 278. An air space is formed between the outer and inner sheets 279 and 280 within the opening 278A of the frame 278. The frame 278 has a drain pipe 281 which is connected with an aperture 282 formed in the frame 278 communicating with the air space between the outer and inner sheet 279 and 280. The lid 274 is mounted on the casing 271 at the opening thereof with the inner flexible sheet 280 spaced a short distance from the surface of the cooling element 272.

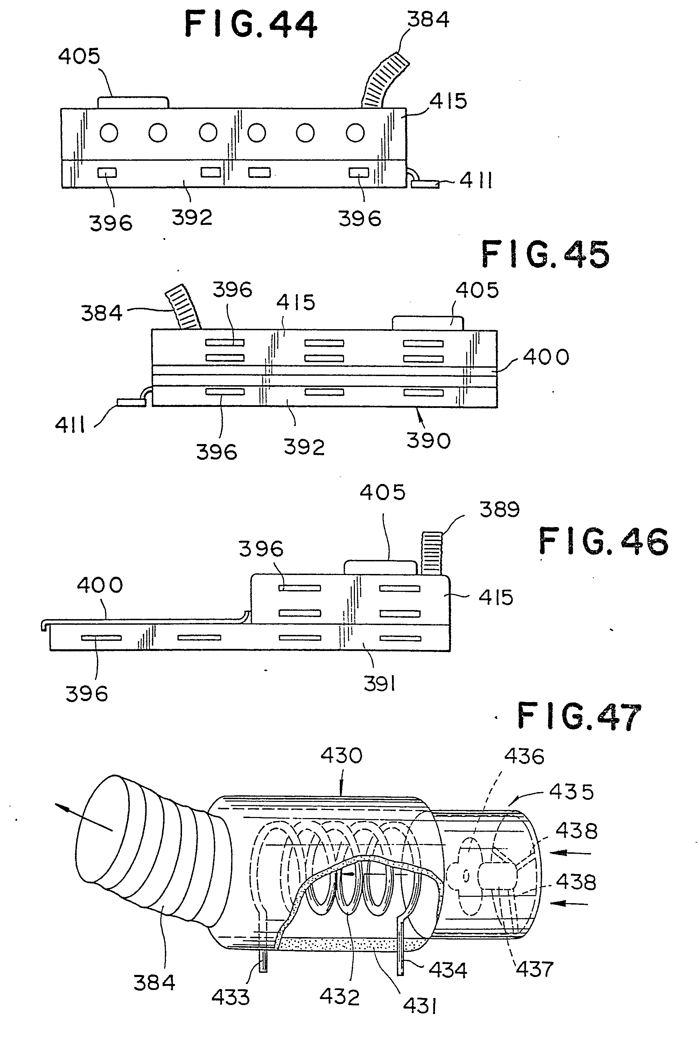

The head rest 270 is mounted on the top of a back 284 in a chair (not shown) as seen from Figure 29.    When a person 285 bears against the closure member 274 of the head rest 270, as shown by a broken line in Figure 44, the outer and inner flexible sheets 279 and 280 are urged inwardly with the inner sheet engaged by the surface of the cooling  element 272 to cool the head of the person 285.

At rest the cooling element 272 can be thermally insulated by an air space in the frame 278 and the air layer between the inner sheet 280 and the surface of the cooling element 272 without condensation of water on the outer surface of the closure member 274.    The sheets can be a flexible sheet material having low-temperature resistance up to about – 20°C and heat conduction, such as silicon rubber sheet containing aluminium powder, polyester film or metal sheet; non-flexible sheet materials can be used if they have these characteristics when the frame 278 will be formed of a deformable cushion material such as polyurethane foam or the like.


Figure 30 shows a device comprising a theremoelement 290 mounted on an open-ended frame 291 of thermally insulated material and having a cold section 292 and a hot section 293.    A lid 294 similar to the closure members 274 and 274A is mounted on the frame 291 at the opening thereof opposite the cold section 292 of the theremoelement 290.  The lid includes a frame 295, an outer flexible sheet 296 bonded to the outer face of the
frame 295, and an inner flexible sheet 297 connected to
the inner face of the frame.   When pressure is applied
to the flexible sheets 296 and 297 as shown by an arrow
298, these sheets are deformed inwardly to engage the
inner sheet 297 with the cold section 292 of the thermoelement 290.

    Figures 21 and 32 show an embodiment in which a
cooling element 300 is made of a sheet material reflecting
alpha waves from the human brain 301 back to the head as
shown by an arrow 304.   This sheet e.g. aluminium sheet,
is incorporated in a head rest 302 of a chair 303.   The
cooling element 300 comprises a plate 305 formed with a
passage 306 for circulating a coolant such as FREON.   The
cooling element may be made of a polyester film having
aluminium deposited on one side or a silicon rubber sheet
containing aluminium powder.   The alpha wave reflecting
and cooling element may be assembled into a pillow which
is used on a bed or mattress.   In another embodiment an
alpha-wave reflecting and cooling plate may be mounted
around the cold section 311 of a thermoelement.

    Figures 33 and 34 show the invention applied to
a person 320 lying on a mattress 321 or a bed.   A cooling
and warming circuit comprises a compressor 322 received in a housing 323 and driven by any suitable means such as
electric motor and a condenser 324 consisting of a
convoluted heat-radiation pipe which receives a
compressed coolant such as FREON from the compressor
322 and also housed in the housing 323.   An evaporator
325 is mounted in a pillow 326 and comprises a cooling
element which receives the coolant from the condenser
324 through a capillary pipe 327.   A blower 328 is
mounted on the top of the housing 323 for feeding air
heated by contacting with the condenser 324 to a nozzle
329 placed at the feet of the lying person 320 through a
flexible hose 330.   The person 320 lying on the mattress
321 will be cooled at his head and warmed at his feet.
In a modification a thermoelement 340 is housed in a
pillow 325 with a cold section disposed against a pillow
portion which is engaged by the head of a person lying on
a mattress.   The hot section of the thermoelement is
connected to a blower placed adjacent to the feet of the
person lying on the mattress.

Figures 35 and 36 show an embodiment in which
the cooling element or evaporator 325A is mounted in the
pillow 326A and comprises a cooling plate 350 of thinner
sheet material having a coolant passage 351.   This
cooling element provides a soothing sound like the murmur
of a brook which is produced when the coolant from the condenser 324A is evaporated at the evaporator 325A.   A
person 320A lying on the mattress 321A can be cooled at his
head by the cooling element 325A and at the same time
mentally calmed by the sound from the interior of the
pillow 326A.

The chair 360 of Figure 37 comprises a head rest
361 having the cooling element 325B, a housing 362 located
between legs 363 of the chair 360 below the seat 364
thereof and receiving a compressor 322B which is connected
to the cooling element 325B in the head rest 361 through a
pipe 365, and a foot rest 366 extending outwardly from the
forward legs 363 and receiving a condenser 324B which is
connected to the compressor 322B.

Figure 38 shows a pillow 370 comprising a head-
engaging hollow portion 371, a pump 372 mounted in the
pillow 370 and connected to the hollow portion 371 through
pipe portions 373 and 374, and a motor 375 disposed in the
pillow 370 for driving the pump 372.   Liquid such as
water is circulated from the pump 374 to the hollow portion
371 through the pipe portions 373 and 374 so that a person
using this pillow 370 will be cooled at his head by water
and at the same time mentally calmed by a sound like the
murmur of a brook which is produced by the circulating
water.

Figures 39 to 46 show a desk of this invention.
A cooling and warming apparatus 380 is placed below a
desk 381 and comprises a foot-rest portion 382 on which
the feet of a person 383 sitting at the desk 381 are
placed, and a flexible hose 384 attached at one end to the
body of the apparatus 380 and extending therefrom near the
forehead of the person 383.   The flexible hose 384 has a
blowing nozzle 385 mounted on the outlet thereof and is
supported in its operative position by means of an articulated support 386 which is mounted on the edge of the
desk 381 by any suitable means such as vise 387.   The
support 386 includes a power switch 386A mounted thereon
at the upper end.

The apparatus 380 comprises a base 390 having
upstanding side and end walls 391 and 392.   Each of the
side walls 391 has a forward wall portion 393 and a
higher rearward wall portion 395.   Vanes 396 in the
walls extend outwardly and downwardly to form downwardly
directed openings for drawing air into the apparatus 380.
Reinforcing members 397 (three shown in Figure 41) are
welded to the inner surface of the forward portion 390A.
The outermost members 397 include threaded holes 398 which
correspond to apertures 399 formed in a foot-rest plate
400.   The plate 400 can be mounted on the base 390 by
means of screws (not shown) extending through the apertures
399 and screwed into the threaded holes 398 to close the forward portion 390A of the base 390.    Between the foot-
rest plate 400 and the reinforcing members 397 is mounted
a heat-radiation pipe 401 as described below.

The rearward portion 390B of the base 390
includes stud bolts 402 disposed on the inner surface
thereof adjacent to one side.    These stud bolts 402 are
received in holes 403 formed in a compressor base plate
404 on which a compressor 405 is mounted, and held there
by nuts (not shown).    The rearward portion 390B also
includes a cooling box 406 mounted on the inner surface
thereof adjacent to the opposite side.    The cooling box
406 is made of any thermally insulating material such as
foamed polystyrene and receives a cooling element 407
connected to the compressor 405 by pipe 408.    Vanes 396
in the walls extend outwardly and downwardly to form
downwardly directed openings for drawing air into the
apparatus 380.    Reinforcing members 397 (three shown in
Figure 41) are welded to the inner surface of the forward
portion 390A.    The outermost members 397 include threaded
holes 398 which correspond to apertures 399 formed in a
foot-rest plate 400.    The plate 400 can be mounted on the
base 390 by means of screws (not shown) extending through
the apertures 399 and screwed into the threaded holes 398
to close the forward portion 390A of the base 390.
Between the foot-rest plate 400 and the reinforcing members 397 is mounted a heat-radiation pipe 401 as described below.  The heat-radiation pipe 401 and cooling plate 407 serve, respectively, as a condenser and evaporator as in the aforementioned cooling and warming device utilising a coolant such as FREON.  A drain pipe 410 is attached to the cooling box 406 to remove droplets condensed on the surface of the cooling plate 407. The drain pipe 410 is received in a water receiver for accumulating the removed water which is disposed outside the base 390.

The cooling box 406 includes a relatively large opening 412 formed in the rearward wall thereof.  A blower 413 e.g. a SILOCCO type, is disposed between the rearward wall of the cooling box 406 and the rearward end wall of the base 390 with the inlet 414 thereof connected to the opening 412 of the cooling box 406.

The rearward portion 390B of the base 390 is closed by a covering 415 having a plurality of vanes 416 similar to those of the base 390.  The covering 415 includes an opening 417 formed therein at the top from which the head of the compressor 405 extends outwardly when the covering 415 is mounted on the base 390.  The covering 415 also includes a relatively small opening 418 formed therein at the top through which the flexible hose 384 (Figures 39 and 36) extends into the base 390 and is connected with the outlet 419 of the blower 413.

When the compressor 405 is driven by any suitable means such as an electric motor housed therein, the foot-rest plate 400 is warmed by the heat-radiation pipe 401 while the forehead of the person 383 (Figures 39 and 40) is cooled by air which is cooled at the cooling plate 407 and supplied therefrom by the blower 413 through the flexible pipe 384 for blowing against the forehead of the person.

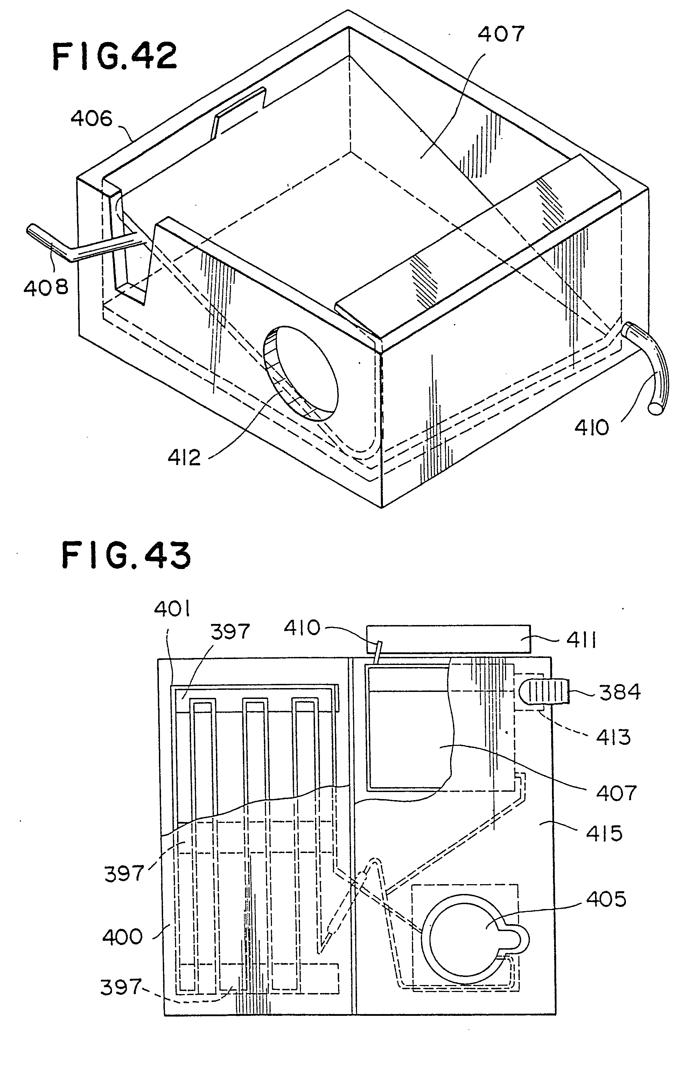

Figure 47 shows another form of the cooled-air supplying means which can be used instead of the cooling box 406 and blower 413 shown in Figures 41 and 42. This comprises a cylindrical hollow casing 430 having a thermally insulating cover 431. Within the casing 430 is disposed a coiled cooling pipe 432 similar to the cooling plate 407 shown in Figures 61 and 62. The cooling pipe 432 includes an inlet and outlet 433, 434 which extend outwardly through the wall of the casing 430 for connecting to the compressor 405 shown in Figures 41 and 43.

A fan device 435 is mounted on the casing 430 at the rearward open end thereof. The fan device 435 comprises a hollow tube in which a fan 436 is coaxially
disposed and connected with an electric motor 437 which
is supported by means of rods 438 in the tube.   When the
fan 436 is driven by the electric motor 437, air is fed
from atmosphere through the casing 430 to the flexible
hose 384.   The air is cooled at the coiled cooling pipe
432 and then conducted into the flexible hose 382.

        This invention provides in Figures 48 to 52
apparatus for measuring the brain power of a person,
which is increased in brain activity by use of apparatus
as in the earlier Figures.   The measuring apparatus
comprises a housing 440 having a front panel 441.   The
front panel 441 includes a finger-socket 442 having a
protective gasket 443.   The front panel 441 includes a
meter 444 having a scale 445 with a needle 446, a tuning
knob 447 for roughly adjusting the needle 446 on the
scale 445, a fine tuning knob 448, a power switch 449,
a display lamp 450 indication power-on, a switch 451 for
controlling a source of light and a display lamp 452 for
indicating light-on.   Within the housing 440 are mounted
a fuse 453 for the power source, a power line 454 and an
attaching plug 455, a power transformer 456, a rectifier
457, an amplifier 458 and a measuring section 459.   The
measuring section 459 includes a housing 460 located
behind the finger-socket 442 of the panel 441.   Referring to Figures 50 and 51, the housing 460 includes upper and lower bridging bars 461 and 462.    The lower bar 462 supports a trough-like finger socket 463 for a human finger 464.    The lower bar 462 also supports a stopper 465 for positioning the finger 464 at a predetermined location in the finger receiver 463.    The upper bar 461 supports a vertical transparent acrylic rod or optical fibre 466 and the lower bar 462 supports another such transparent rod 467 in vertical alignment with the upper rod 466.    A lamp light source 468 is mounted on the housing 460 above the supper transparent rod 466, and has a top reflector 469.    A photoreceptor 470 is mounted near the lower transparent rod 467.

When the finger 464 is received in the finger receiver 463 and the lamp 468 is turned on, light is transmitted along the upper transparent rod 466, through the positioned finger 464 and along the lower transparent rod 467 to the photoreceptor 470.    The blood vessels in the finger 464 are connected to the brain 471 through vessels 472; if the flow of blood in the brain 471 is changed the flow of blood in the finger 464 also is correspondingly changed and in this way a change in brain activity can be detected by measuring the flow of blood in the finger 464 which will appear at the photoreceptor 470 as any change of photocurrent.

In use, the apparatus works as shown in the circuit diagram of Figure 52.   The power switch 449 is switched on, the finger 464 is inserted into the receiver 463 through the socket 442 until it engages the stopper 465.   The light switch 451 is turned on and then the adjusting knobs 447 and 448 are moved to obtain zero or like base indication of the indicator 446 in the meter 444.  The finger 464 is then rotated around its central axis until the needle 446 shows the minimum value.   If the needle 446 is moved beyond the maximum value of the scale 445 the needle 446 is brought within the scale 445 by adjusting the knobs  447 and 448.   The light switch 451 is turned off and the finger 464 is removed from the opening 442.   The person under test is subjected to the operation of the apparatus previously described, his finger 464 is again inserted into the finger receiver 463 and the light switch 451 is turned on to measure a measurement in the meter 444 without movement of the adjusting knobs 447 and 448.   This measurement is compared with the previous measurement to measure an increase of brain power. . Evaluations were done using the performance test paper shown in Figure 53.   This paper has four sets of randomly arranged figures, each set consisting of two lines each having sixty-three numerals.   In use, the sum of two digits obtained by multiplying adjacent figures in each line is mentally
calculated by the person under test, the digits are
added together and his answer is written on the paper.
For example, a first line in a first step is calculated
as follows:

$$3 \times 4 = 12, \quad 1 + 2 = \underline{3};$$
$$4 \times 5 = 20, \quad 2 + 0 = \underline{2};$$
$$5 \times 9 = 45, \quad 4 + 5 = \underline{9};$$

........................

The figures 3, 2, 9 .... . which are underlined, are
written between each adjacent figures above the first line
of the first set.   This calculation is repeated for each
line of each set.   After two minutes elapse, the
calculation is changed from one set to another.   When all
the calculations have been done, the correct and false
answers are counted.   The correct answers are used to
estimate a performance of operation for a particular tested
person.   The results obtained in evaluations using six
different students each of whom was subjected to treatment
for 10 to 20 minutes, the head being cooled to about 5°C
and the feet warmed to about 40°C are shown in Table 1.
These results show that following use of the apparatus
the subjects tested improved their scores and that the
brain activity meter of the invention produced results
corresponding to the increase in the recorded answers.

| SUBJECT | Before use of the apparatus | | After use of the apparatus | | Change | Measurement | | |
|---|---|---|---|---|---|---|---|---|
| | Correct Answers | False Answers | Correct Answers | False Answers | Improvement | Before | After | Difference |
| Student KK | 303 | 3 | 392 | 3 | 29% | 24 | 31 | 29% |
| Student MS | 249 | 10 | 309 | 4 | 24% | 33 | 43 | 30% |
| Student MS | 151 | 7 | 182 | 2 | 20% | 9.5 | 16 | 68% |
| Student T | 224 | 8 | 285 | 3 | 34% | 32 | 36 | 13% |
| Student I | 206 | 2 | 275 | 1 | 33% | 20 | 18 | 11% |
| Student S | 179 | 0 | 240 | 0 | 34% | 33 | 27 | 22% |

## CLAIMS

1.   Apparatus for increasing the mental activity of a human person characterised by means for cooling the head of the person and means for warming the sole of the person.

2.   Apparatus according to Claim 1, characterised in that it is incorporated in an article of furniture.

3.   Apparatus according to Claim 1 or 2, characterised in that the apparatus is incorporated in a chair, with the cooling means being mounted at or near a head rest and the warming means being mounted at or near a foot rest.

4.   Apparatus according to any preceding Claim, characterised in that the cooling and warming means comprises a compressor for compressing a coolant, a condenser connected to the compressor for receiving the compressed coolant and radiating heat, and an evaporator connected with the condenser through a pipe for evaporating the coolant from the condenser and for returning the evaporated coolant to the condenser, the cooling means including the evaporator and said warming means including the condenser.

5.   Apparatus according to Claim 4, characterised in that a blower means is connected with the evaporator for blowing air cooled at the evaporator toward the head of the said person sitting in the chair.

6.   Apparatus according to Claim 4 or 5, characterised in that the evaporator is housed in the head rest of the chair including a thermally insulating yieldable member adapted to yield under the pressure of the head of the person.

7.   Apparatus according to Claim 6, characterised in that the thermally insulating yieldable member includes at least one flexible sheet spaced from the evaporator to form an air gap therebetween.

8.   Apparatus according to any preceding Claim, characterised in that the cooling and warming means includes an electric fan means housed in the chair, electric heating means in the foot rest, and wherein the head rest includes a plurality of openings for drawing air into the interior of the head rest and then blowing air towards the head of the person when the fan means is energised.

9.   Apparatus according to Claim 1, 2 or 3, characterised in that the cooling and warming means comprises a thermoelement utilising the Peltier effect with the cold section located in a head rest and the hot section disposed in a foot rest.

10. Apparatus according to Claim 9, <u>characterised in that</u> the cooling and warming means is mounted in a head pillow and has a cold section to be engaged by the head of a person using the pillow and a hot section connected with blower means via a conduit and including a nozzle adapted to be placed adjacent to the feet of the user.

11. Apparatus according to Claim 1, 2 or 3, <u>characterised in that</u> the cooling and warming means comprises a housing containing a compressor for compressing a coolant and a condenser for receiving the compressed coolant and radiating heat, and blower means for receiving air heated at the condenser and feeding the heated air to another nozzle via flexible duct means, and an evaporator disposed in a head pillow and connected with the condenser through a capillary pipe for evaporating the coolant from the condenser.

12. Apparatus according to Claim 1 or 2, <u>characterised in that</u> the cooling and warming means comprises a housing having a foot rest and containing a compressor for compressing a coolant and a condenser adjacent the foot rest for receiving the compressed coolant from the compressor and an evaporator connected with the condenser through a capillary pipe for evaporating the coolant from the condenser and connected with said compressor for returning the coolant thereto, and duct means extending outwardly of the housing and connected to the evaporator at the inlet of the duct means, and blower means positioned between the evaporator and the duct inlet for drawing in and blowing out air cooled at the evaporator.

13. Apparatus according to any one of Claims 1 to 12, characterised in that the cooling means is disposed at the head rest of a seat in a vehicle and the warming means comprises the vehicle heater.

14. Apparatus according to any one of Claims 1 to 12, characterised in that it is incorporated in a student's desk.

15. Apparatus according to any preceding Claim, characterised in that the evaporator comprises a cooling plate made of a material adapted to reflect alpha waves from the human brain.

16. Apparatus according to any preceding Claim, characterised in that the evaporator is shaped and adapted to produce a soothing noise.

17. Apparatus for measuring the activity of the human brain, characterised by means for receiving a part of the body of a person whose brain power is to be measured, means for radiating light through the body part, means for receiving the light transmitted through the body part and converting it into an electrical signal, and means for indicating the magnitude of said electrical signal.

18. Apparatus according to Claim 17, characterised in that the body part has blood vessels and is e.g. a finger.

FIG.3

FIG.4

FIG.1

FIG.2

# FIG.5

64 63 62 61 22 33 60

65

64

50

# FIG.6

22

67

66

33

60A

50

68

# FIG.7

33 60B 22

71

70

50

# FIG.8

76

79

81

80 78

22

33

60D

50

# FIG.9

22

83

82

60E

33A

50

# FIG.10

92A 92

91
90
94
94
93A
93
95
96
97A 97

# FIG.11

92 92A
91
94
90
93A
93B
97
96
97B
97A
95

# FIG.12

92
91
90
93
93C
96
100
97

# FIG.13

116
117
118
113
111
114 115 110 112

FIG.15

FIG.14

FIG.16

FIG.17

FIG.18

FIG.19

FIG.20

FIG.21

FIG.22

FIG.23

FIG.24

# FIG.25

A

QUANTITY OF HEAT

AS

PE

DISTANCE

# FIG.26

RECOVERY

AS

PG

TEMPERATURE (-°C)

# FIG.27

265
261 264 263
260

262

# FIG.28

274

279
278A
278
280

272
271

281

273

270

277

276

275

# FIG.29

285

279
280

272
271
273

270

284

278

# FIG.30

294
296 298 291

295

297 293 292
290

FIG.31

FIG.32

FIG.33

FIG.34

FIG.35

321A  326A  327A  323A  324A  322A  321A  325A

FIG.36

351  350

FIG.37

361  325B  365  364  360  324B  363  366  322B  363

FIG.38

371  370  373  372  374  375

FIG.39

385

386A

383

384

386

387

381

382    396    380

FIG.40

386

383

387

384

381

380

396    396    411

# FIG.41

FIG.42

406

407

408

412

410

FIG.43

401

397

410

411

384

413

407

415

397

400

405

397

0061843

12/14

**FIG.44**

405      384

415

411

396     392     396

**FIG.45**

384    396    415     405

400

411

396     392     390

405      389

396     **FIG.46**

400     415

396     391

**FIG.47**

430     436     435

438

438

437

384   433   432   431   434

# FIG.48

# FIG.49

# FIG.50

# FIG.51

# FIG.52

455 454 449 453 456 457

450

451

452

447 448

460

468 466 464 467 470 458 444

AMP

∇
E

# FIG.53

| | | | | | | | | | | | | | |
|---|---|---|---|---|---|---|---|---|---|---|---|---|---|
| ① | 3 | 4 | 5 | 9 | 9 | 6 | 3 | | 8 | 6 | 9 | 2 | 5 | 4 | 6 |
| | 5 | 6 | 3 | 9 | 4 | 6 | 8 | | 6 | 4 | 9 | 9 | 7 | 3 | 6 |
| ② | 8 | 4 | 6 | 6 | 9 | 7 | 3 | | 7 | 9 | 8 | 7 | 3 | 5 | 6 |
| | 6 | 3 | 9 | 4 | 8 | 3 | 7 | | 3 | 9 | 5 | 5 | 6 | 5 | 3 |
| ③ | 3 | 9 | 7 | 4 | 8 | 2 | 5 | | 9 | 5 | 5 | 3 | 8 | 4 | 6 |
| | 8 | 5 | 2 | 5 | 3 | 4 | 7 | | 6 | 8 | 3 | 9 | 9 | 6 | 5 |
| ④ | 5 | 9 | 3 | 5 | 2 | 8 | 6 | | 6 | 4 | 3 | 7 | 4 | 9 | 2 |
| | 2 | 5 | 4 | 7 | 8 | 5 | 6 | | 2 | 9 | 5 | 6 | 8 | 9 | 5 |